# EUROPEAN PATENT APPLICATION

(11) **EP 1 362 613 A1**
(43) Date of publication of application: **19.11.2003**
(21) Application number: 02008637.7
(22) Date of filing: 17.04.2002
(51) Int. Cl.: A61N 1/20, A61N 1/30, A61N 1/32

(54) **Device for the resolution of calcific masses**

(71) Applicant: Regazzoni, Giovanni, 20148 Milano (IT); Corrado, Ezio Maria, 80121 Napoli (IT); Russo, Sergio, 80127 Napoli (IT); Bellucci, Francesco, 80127 Napoli (IT)
(72) Inventor: Regazzoni, Giovanni, 20148 Milano (IT); Corrado, Ezio Maria, 80121 Napoli (IT); Russo, Sergio, 80127 Napoli (IT)
(74) Representative: Rapisardi, Mariacristina

(57) **Abstract**

A device (1) for resolving calcific masses (39) comprises a pair of electrodes (12) which can be connected to a power supply (30, 31).

One of the electrodes (12) can be positioned in correspondence with the calcific mass (39), and is suitable for causing the elimination of the calcific mass (39) through an electrolytic process.

## Description

The present invention refers to a device for the resolution of calcific masses.

The device according to the present invention can be used, for example, to treat paraosteopathia, myositis ossificans, periprosthetic calcification and calcification in general, herniated disks, osteophytes and growth processes in general.

In practice, the device according to the invention allows newly formed disease-infected bone tissues to be resolved, that is it absorbs said tissues.

Currently, when a patient has a disease of the type indicated or something similar to it, surgical intervention is carried out.

However, surgical intervention is highly invasive for the patient and, moreover, usually causes numerous other drawbacks.

For example, a surgical intervention usually requires long hospitalisation and rehabilitation periods and it implies numerous risks, which are always present in any case when surgical intervention on the human body is involved, etc.

Surgical operation, in particular when one intervenes on calcific masses of a substantial size (calcific masses can weigh as much as a few kilograms) can require a very long time and the results may also only be partial or temporary.

It is clear that such a situation is extremely demanding and stressful both for the doctors and for the patient.

Moreover, operations of the type indicated are necessarily carried out under general or local anaesthetic.

The technical task proposed of the present invention is, therefore, that of eliminating the aforementioned technical drawbacks of the prior art, by realising a device for resolving calcific masses which allows calcific masses to be resolved with interventions which are not overly invasive.

In this technical task a purpose of the invention is that of realising a device which allows calcific masses, which may even be of a substantial size, to be resolved in a short space of time, avoiding the trauma of surgery.

The last but not least purpose of the invention is that of realising a device for resolving calcific masses which is substantially simple and safe.

The technical task, as well as these and other purposes, according to the present invention, are achieved by realising a device for resolving calcific masses, characterised in that it comprises at least one pair of electrodes which can be connected to power supply means, at least one of said electrodes being positionable in correspondence with said calcific mass and being suitable for causing the elimination of said calcific mass through an electrolytic process.

Other characteristics of the present invention are defined, moreover, in the subsequent claims.

Advantageously, the device according to the present invention allows the hospitalisation and rehabilitation times after the intervention to be substantially reduced.

Moreover, since the operations are very quick, the intervention of many surgeons is not usually required, but a single surgeon manages to complete the whole of the intervention.

In practice, the device according to the invention also allows calcific masses of a large size to be resolved within the space of a few hours.

This means less stressful and demanding interventions as much for the doctors as for the patients.

Finally, the device allows calcific masses to be resolved without requiring that the patient be subjected to any anaesthetic.

Further characteristics and advantages of the invention shall become clearer from the description of a preferred but not exclusive embodiment of the device for resolving calcific masses according to the finding, illustrated for indicating and not limiting purposes in the attached drawings, in which:

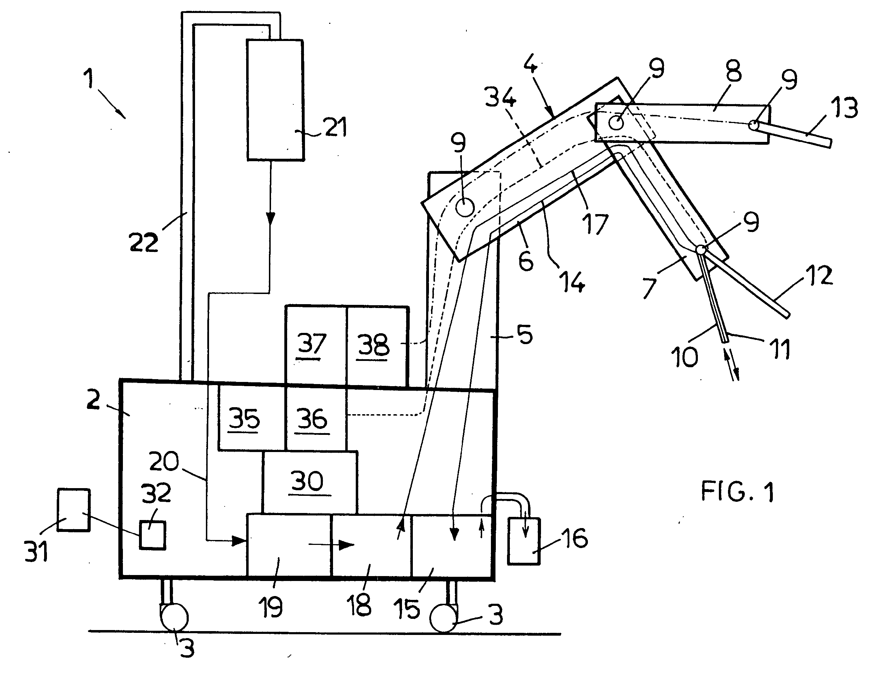

figure 1 shows a schematic side view of a device according to the present invention;

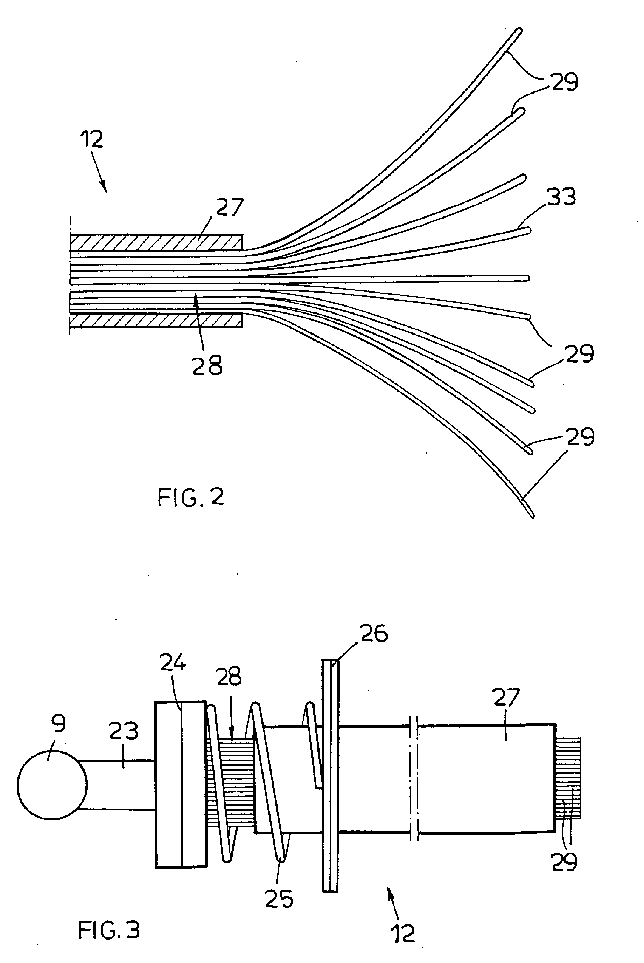

figure 2 shows an enlarged section view of a detail of an electrode according to the invention;
figure 3 shows an electrode of the device according to the invention; and
figures 4a-4d show for different treatment steps of a calcific mass.

With reference to the quoted figures, a device for resolving calcific masses is shown, wholly indicated with reference numeral 1.

The device 1 comprises a structure 2 equipped with wheels 3 which make it easier to be moved.

The structure 2 carries an articulated arm 4, made up of numerous rods 5, 6, 7, 8 connected to each other through lockable joints 9.

By using the joints 9 the surgeon can position the arm 4 and lock it, so as to be able to operate without having to support it.

In the example shown, the two rods 7 and 8 are connected to the rod 6 of the arm 4 through the joint 9.

The rod 7, in turn, is connected through a further joint 9 to two tubes 10, 11, which are, for example, integral with each other, and to an electrode 12 (anode).

The rod 8 is connected, through the joint 9, to an ultrasound scanning probe 13.

The tube 10 is connected, through a duct 14, to an intake group. This, in turn, is connected to a bowl 16 for containing the liquid sucked in, which is outside of the structure 2 and which can be removed and replaced.

The tube 11, on the other hand, is connected through a duct 17 to a cooling group 18 associated with a washing group 19.

The washing group 19 is connected, through a duct 20, to a sac 21 containing a washing liquid supported by an upright 22 integral with the structure 2.

Usually, such a sac 21 consists of a sac of physiological solution with a capacity of 5 litres.

The electrode 12 comprises a rod 23, connected on one side to the joint 9 and equipped on the opposite side with a flange 24.

An elastic member, such as a spring 25, is connected to the flange 24, said elastic member being fixed to a second flange 26 integral with a tubular element 27.

Inside the tubular element 27 a conductor 28 is capable of sliding, said conductor consisting of a plurality of wiry elements 29 with the ends associated with the flange 24.

The wiry elements 29 are realised in shape memory material and are coated through an insulating material, for example Teflon or paraffin, apart from the end portion.

As is known, in shape memory materials a certain shape can be set which is taken up by them at a given temperature.

In this way when the electrode 12 is not used the elements 29 are housed inside the element 27 and are rectilinear, and when, on the other hand, the conductor 28 is removed from the element 27, the elements 29 heat up (the temperature increases up to human body temperature) and they once again take up the shape with which they were preset.

Advantageously, the elements 29 have a curvilinear preset shape. Moreover, the elements 29 are inserted inside the tubular element 27 so that, when partially removed, their free ends tend to move away from each other and the wiry elements 29 take up an umbrella arrangement.

The spring 25 constitutes control means which control the release of the conductor 28 from the tubular element 27.

In a different embodiment (not shown) the control means comprise an actuator commanded by an electronic processor.

Of course, the conductor 28 is connected to power supply means which usually comprise a battery 30 and/or the mains 31 associated with a rectifier 32.

Moreover, optical fibres 33 are also inserted so that they can slide inside the tubular element 27, said fibres being connected, through cables 34, to a lighting device 35 of the optical fibres 33, to a device 36 for recording the images taken by the optical fibres themselves 33 and to a screen 37.

The optical fibres 33 and the devices 35, 36 connected to them constitute fibre-optic visualisation means and allow blood vessels, nerves and other body parts to be detected and allow the advance of the electrolytic process to be visualised.

The ultrasound scanning probe 13 is connected to an ultrasound scanning device 38, in turn connected to the screen 37.

The device 1 also comprises a second electrode (cathode, not shown) necessary to complete the circuit, which is electrically connected to the anode and which can be positioned either close to the anode, in correspondence with the calcific mass, or distanced from the anode; in the latter case, for example, the cathode can be introduced into a water-filled container.

The operation of the device for resolving calcific masses according to the invention can be seen clearly from that which has been described and illustrated and, in particular, is substantially the following.

Beforehand, through a radioscopic device (not forming part of the device 1), the position and consistency of the calcific mass is measured.

Alternatively, the ultrasound scanning probe 13 (of the known type) is applied and then the electrode 12 is inserted into contact with the calcific mass to be resolved and, in correspondence with the calcific mass itself, the tubes 10, 11 are also inserted.

Finally, the device 1 is put into action, sending power to the wiry elements 29 and starting up the washing group 19, cooling group 18 and intake group 15 (the arrows indicate the flow direction of the liquid).

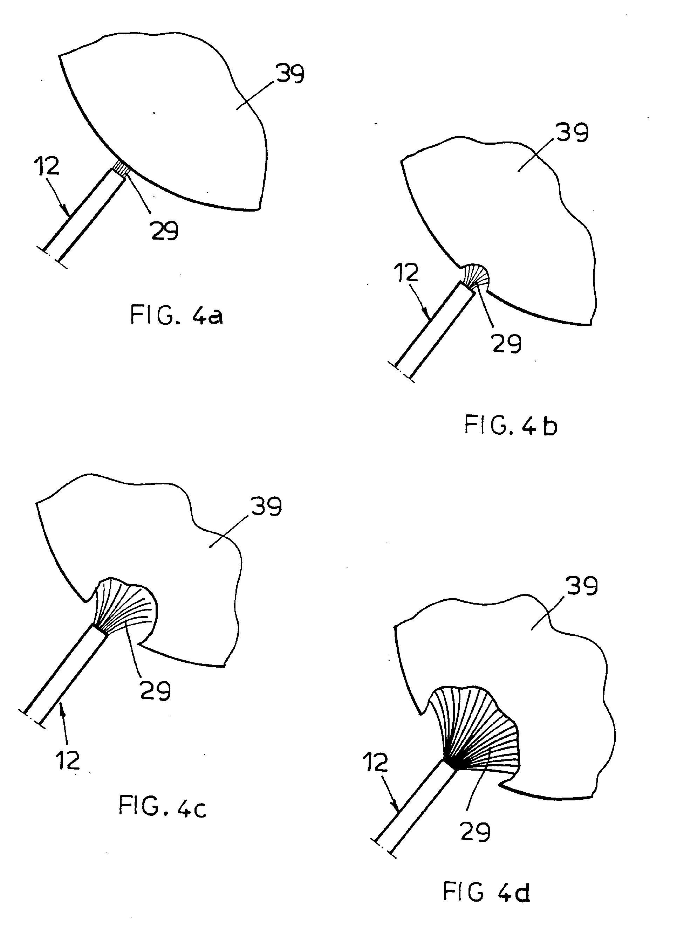

As shown in figures 4a-4d, the wiry elements 29 are initially contained inside the tubular element 27.

The passage of the current through the wiry elements 29 sets off an electrolysis process which uses the wiry elements 29, the calcific mass 39 and the interstitial fluids and/or the liquid or physiological solution.

The electrolysis causes the passage into solution of Ca⁺⁺ ions and, therefore, the resolution of the calcific mass 39.

However, the resolution speed of the calcific mass 39 is directly proportional to the intensity of the current which crosses the wiry elements 29.

Therefore, to obtain a high resolution speed of calcific masses, with the device 1 according to the invention high currents, with intensity in the order of one or more amps (direct current), are used.

The spring 25 pushes the conductor 28 and, therefore, the wiry elements 29 against the calcific mass 39. In this way even if the size of the calcific mass 39 decreases as the process progresses, the wiry elements 29 are always in contact with the calcific mass 39.

Moreover, coming out from the tubular element 27, the wiry elements 29 tend to widen or expand, engaging increasing portions of the calcific mass 39 as the process progresses.

In this way calcific masses 39 of a substantial size are also treated in reduced periods of time without it being necessary to treat all of the calcific mass 39 to be resolved one bit at a time.

Moreover, as the electrolytic process progresses, the washing group 19, through the tube 11, injects the physiological solution, which has been previously cooled by the cooling group 18, into the body of the patient.

At the same time, the intake group 15, through the tube 10 and the duct 14, takes in the liquid containing the calcium ions which have been removed from the calcific mass 39 and collects it in the bowl 16.

Advantageously, the cooled liquid which is introduced into the body through the duct 11 in correspondence with the calcific mass 39, cools all of the elements with which it comes into contact. Then, such a liquid and the heat which it has accumulated are removed from the duct 10.

In this way one also manages to operate with high-intensity currents (equal to or greater than one amp in direct current), without localised heating being caused which would be very harmful to the patient.

Modifications and variants, besides those already mentioned, are of course possible, for example, in different embodiments the device has no washing, cooling and intake groups (for treating calcific masses of a very small size) or else it can have no ultrasound scanning probe.

In practice, it has been noted how the device according to the invention is particularly advantageous since it allows calcific masses even of a substantial size to be resolved with scarcely invasive interventions and in very short periods of time.

Moreover, the device is very safe and, in particular, even operating with high currents, does not cause harmful localised heating.

The device for resolving calcific masses thus conceived is susceptible to numerous modifications and variants, all covered by the inventive concept; moreover, all of the details can be replaced by technically equivalent elements.

In practice, the materials used, as well as the sizes, can be whatever according to the requirements and the state of the art.

## Claims

1. Device (1) for resolving calcific masses (39), **characterised in that** it comprises at least one pair of electrodes (12)which can be connected to power supply means (30, 31), at least one of said electrodes (12) being positionable in correspondence with said calcific mass (39) and being suitable for causing the elimination of said calcific mass (39) through an electrolytic process.

2. Device (1) according to claim 1, **characterised in that** said electrode (12) comprises a conductor (28) slidably inserted inside a tubular element (27).

3. Device (1) according to one or more of the previous claims, **characterised in that** said conductor (28) is associated with control means (25) which control the release thereof from said tubular element (27).

4. Device (1) according to one or more of the previous claims, **characterised in that** said control means (25) comprise elastic members.

5. Device (1) according to one or more of the previous claims, **characterised in that** said control means comprise an actuator commanded by an electronic processor.

6. Device (1) according to one or more of the previous claims, **characterised in that** said conductor (28) comprises a plurality of wiry elements (29).

7. Device (1) according to one or more of the previous claims, **characterised in that** said wiry elements (29) are realised in shape memory material, so that when said wiry elements (29) come out from said tubular element (27) they expand.

8. Device (1) according to one or more of the previous claims, **characterised in that** each of said wiry elements (29) is coated with an insulating material.

9. Device (1) according to one or more of the previous claims, **characterised in that** inside said tubular element (27), together with said wiry elements (29), fibre-optic visualisation means (33, 35, 36) are slidably associated.

10. Device (1) according to one or more of the previous claims, **characterised in that** said electrode (12) is supported at the end by an articulated arm (4) which extends from a structure (2) of said device (1).

11. Device (1) according to one or more of the previous claims, **characterised in that** said articulated arm (4) has at least one lockable joint (9).

12. Device (1) according to one or more of the previous claims, **characterised in that** said device (1) also comprises a washing group (19) and an intake group (15), suitable for introducing a liquid near to the treated zone and for removing another liquid containing a material removed from said calcific mass (39) from said treated zone.

13. Device (1) according to one or more of the previous claims, **characterised in that**, associated with said washing group (19), said device (1) also comprises a cooling group (18) of said liquid to be introduced near to the treated zone.

14. Device (1) according to one or more of the previous claims, **characterised in that** it comprises an ultrasound scanning probe (13).

15. Procedure for resolving calcific masses (39), **characterised in that** an electrode (12) is introduced in correspondence with said calcific mass (39) to be treated and direct current is made to pass through it so that, by electrolysis, calcium ions free themselves from said calcific mass (39) which is eliminated.

16. Procedure according to claim 15, **characterised in that**, in correspondence with said calcific mass (39), a washing liquid is introduced in which the material, which has been eliminated from said calcific mass (39), passes, said liquid being subsequently removed together with said material eliminated from said calcific mass (39).

17. Procedure according to claim 16, **characterised in that** said washing liquid is cooled before being introduced in correspondence with said calcific mass (39).

18. Device (1) for resolving calcific masses (39) as described and claimed.
